# EUROPEAN PATENT APPLICATION

(11) **EP 4 524 257 A1**
(43) Date of publication of application: **19.03.2025**
(21) Application number: 23803766.7
(22) Date of filing: 04.05.2023
(51) Int. Cl.: C12Q 1/6806, C12Q 1/6844, C12Q 1/70

(54) **METHOD FOR PREPARING REACTION MIXTURE FOR NUCLEIC ACID AMPLIFICATION REACTION IN SAMPLE BY USING SAMPLE HANDLING SYSTEM**

(30) Priority: 13.05.2022 KR 20220059077
(71) Applicant: Seegene, Inc., Seoul 05548 (KR)
(72) Inventor: CHOI, Jinhwan, Namyangju-si Gyeonggi-do 12248 (KR); HAN, Jooyeon, Seoul 05545 (KR); KIM, Seong-Su, Uijeongbu-si Gyeonggi-do 11803 (KR)
(74) Representative: Engelhard, Markus
(86) International application number: PCT/KR2023/006141
(87) International publication number: WO 2023/219343

(57) **Abstract**

The present invention relates to a method for preparing reaction mixtures for amplifying nucleic acids present in samples using a sample handling system. Specifically, by utilizing two types of pipetting mechanisms and specific nucleic acid extraction reagents, the time required to extract nucleic acids from samples and mix them with amplification reagents can be significantly reduced, thereby greatly enhancing the efficiency of molecular diagnostics based on nucleic acid amplification reactions.

## Description

### [Technical field]

The present invention relates to a method for preparing reaction mixtures for amplifying nucleic acids present in samples using a sample handling system.

### [Background Art]

Since the global pandemic of SARS-COV-2, there has been a rapid increase in demand for technologies capable of diagnosing pathogen infections, such as bacteria and viruses in samples collected from suspected patients. Given the nature of diagnostic technologies, ensuring high accuracy in diagnostic results is of utmost importance, so molecular diagnostic methods using nucleic acid amplification principles are generally employed.

Suggested for nucleic acid amplification has been a variety of methods including PCR (polymerase chain reaction), strand displacement amplification (SDA, B. J. Toley, I. Covelli, Y. Belousov, S. Ramachandran, E. Kline, N. Scarr, N. Vermeulen, W. Mahoney, B. R. Lutz and P. Yager, Analyst, 2015, 140, 7540-7549)), helicase dependent amplification (HAD, M. Vincent, Y. Xu and H. Kong, EMBO Rep., 2004, 5, 795-800), recombinase polymerase amplification (RPA, J. Li, J. Macdonald and F. von Stetten, Analyst, 2018, 144, 31-67), loop-mediated isothermal amplification (LAMP, Y. Mori, H. Kanda and T. Notomi, J. Infect. Chemother., 2013, 19, 404-411), RCA (Rolling Circle Amplification, M. M. Ali, F. Li, Z. Zhang, K. Zhang, D.-K. Kang, J. A. Ankrum, X. C. Le and W. Zhao, Chem. Soc. Rev., 2014, 43, 3324-3341.), TMA (Transcription-Mediated Amplification, see L. Comanor, Am. J. Gastroenterol., 2001, 96, 2968-2972), NASBA (nucleic acid sequence-based amplification, A. Borst, J. Verhoef, E. Boel and A. C. Fluit, Clin. Lab., 2002, 48, 487-492), or reverse transcription polymerase chain reaction (RT-PCR).

The most predominant process for a nucleic acid amplification known as polymerase chain reaction (hereinafter referred to as "PCR") is based on repeated cycles of denaturation of double-stranded DNA, followed by oligonucleotide primer annealing to the DNA template, and primer extension by a DNA polymerase (Mullis et al. U.S. Pat. Nos. 4,683,195, 4,683,202, and 4,800,159; Saiki et al., (1985) Science 230, 1350-1354).

However, current molecular diagnostics systems require a long time to confirm results and must be operated by skilled experts, making it difficult to conduct multiple tests simultaneously in locations other than laboratories and specialized institutions. Therefore, in order to perform the molecular diagnostics regardless of time and place, it is necessary to reduce the time required for each step of the complex nucleic acid amplification, or to overcome problems such as contamination caused by operator errors by automating most of the procedures.

Molecular diagnostics using a nucleic acid amplification reaction, nucleic acids containing the genetic information of pathogens suspected to cause disease are generally extracted from the sample prior to amplification. Using conventional methods, it takes about 1 to 2 hours just to extract nucleic acids from a sample, and several steps are required, such as collecting the extracted nucleic acids, mixing the with nucleic acid amplification reagents, and dispensing them before starting the amplification phase.

Therefore, reducing the time and cost during the setup process prior to initiating the nucleic acid amplification reaction in conventional molecular diagnostic methods is one of the ways to improve the efficiency of highly accurate molecular diagnostics. Recently, research has been focused on addressing this aspect, but there are still issues such as the high cost of the developed equipment and reagents or reduced detection accuracy.

Accordingly, the present inventors have developed a method for preparing reaction mixtures for amplifying nucleic acids in samples, which reduces the number of steps in the nucleic acid extraction process and decreases the time required for each step by using a specific sample handling system and reagents, thereby enabling accurate detection of target nucleic acids while significantly shortening the turnaround time (TAT).

Throughout this application, various patents and publications are referenced and citations are provided in parentheses. The disclosure of these patents and publications in their entities are hereby incorporated by references into this application in order to more fully describe this invention and the state of the art to which this invention pertains.

### [Disclosure]

### [Technical Problem]

The present inventors have made efforts to reduce the turnaround time for molecular diagnostics using nucleic acid amplification reactions. As a result, we have developed a method for preparing reaction mixtures for amplifying nucleic acids that utilizes a sample handling system with two types of pipetting mechanisms, which significantly reduces the time required for extracting nucleic acids from the sample and mixing them with amplification reagents while maintaining excellent nucleic acid extraction efficiency.

Accordingly, it is object of this invention to provide a method for preparing reaction mixtures for amplifying nucleic acids present in samples using a sample handling system.

It is further object of this invention to provide a sample handling system that can perform the above method.

Other objects and advantages of the present invention will become apparent from the detailed description to follow taken in conjugation with the appended claims and drawings.

### [Technical Solution]

In an aspect of the invention, the present disclosure provides a method for preparing reaction mixtures for amplifying nucleic acids present in samples using a sample handling system,
wherein the sample handling system comprises a first pipetting mechanism including 4 to 8 pipetting channels for aspirating and dispensing metered liquids, and a second pipetting mechanism including 96 pipetting channels for aspirating and dispensing metered liquids, wherein the first pipetting mechanism is configured to move the 4 to 8 pipetting channels independently, and the second pipetting mechanism is configured to move all 96 pipetting channels together, wherein each pipetting channel has a pipette tip mounted or mountable thereon,
wherein the method comprises:
   (a) dispensing a plurality of samples into each well of a first plate having 96 wells using the first pipetting mechanism;
   (b) extracting nucleic acids from the samples by dispensing a nucleic acid extraction reagent into each well of the first plate using the second pipetting mechanism;
   (c) preparing a nucleic acid amplification reagent by combining nucleic acid amplification components, and then dispensing the nucleic acid amplification reagent into each well of a second plate having 96 wells using the first pipetting mechanism; and
   (d) dispensing nucleic acids extracted in step (b) into each well of the second plate by using the second pipetting mechanism.

In an embodiment, the first pipetting mechanism and the second pipetting mechanism are located separately within the sample handling system.

In an embodiment, the first pipetting mechanism is configured to move 4 to 8 pipetting channels in a direction of X-axis simultaneously, and to move them in directions of Y-axis and Z-axis independently.

In an embodiment, wherein the second pipetting mechanism is configured to move 96 pipetting channels in the direction of Z-axis independently.

In an embodiment, the sample handling system comprises a section for mounting the first plate, a section for mounting the second plate a section for nucleic acid extraction, and a section for applying magnetic field.

In an embodiment, the sample handling system further comprises a section for mounting sample vessel, a section for mounting a nucleic acid extraction reagent vessel and a section for mounting a nucleic acid amplification component vessel.

In an embodiment, the sample handling system further comprises a section for mounting pipette tips for the pipetting channels of the first pipetting mechanism, and a section for mounting pipette tips for the pipetting channels of the second pipetting mechanism.

In an embodiment, the sections for mounting pipette tips each comprise a section equipped with pipette tips having a capacity of 200 to 400 µL, and a section equipped with pipette tips having a capacity of 900 to 1,100 µL.

In an embodiment, the sample handling system further comprises plate grippers configured to move the first plate and the second plate from one section to another.

In an embodiment, the plate grippers are mounted on two pipetting channels of the first pipetting mechanism, the two pipetting channels being arranged on opposite sides of the plate respectively such that the plate grippers grip and move the plate.

In an embodiment, in step (a), the internal control (IC) is dispensed using the first pipetting mechanism equipped with a pipette tip having a capacity of 200 to 400 µL.

In an embodiment, the nucleic acid extraction reagent comprises a lysis/binding buffer, a first wash buffer, a second wash buffer, and an elution buffer, each contained in a separate vessel.

In an embodiment, in step (a), the first plate is preloaded with magnetic beads, and the sample is dispensed after mounting the first plate in the section for nucleic acid extraction.

In an embodiment, in step (b), the method comprises:
transferring the first plate to the section for applying magnetic field after dispensing 300 to 500 µL of the lysis/binding buffer into the first plate;
removing the lysate using the second pipetting mechanism after collecting the beads by means of the magnetic field; and
transferring the first plate with the lysate removed to the section for nucleic acid extraction.

In an embodiment, the nucleic acid extraction reagent comprises proteinase K, a lysis buffer, a binding buffer, a first wash buffer, a second wash buffer, a third wash buffer and elution buffer.

In an embodiment, the sample handling system further comprises a section for mounting a magnetic bead vessel.

In an embodiment, in step (a), before dispensing the sample, the first plate is mounted on the section for nucleic acids extraction, followed by dispensing 3 to 20 µL of proteinase K into the first plate.

In an embodiment, the proteinase K is dispensed using the second pipetting mechanism equipped with the pipette tip having a capacity of 200 to 400 µL.

In an embodiment, in step (b), the method comprises:
dispensing 100 to 200 µL of lysis buffer into the first plate;
dispensing 3 to 20 µL of magnetic beads into the first plate;
dispensing 500 to 700 µL of binding buffer, followed by transferring the plate to the section for applying magnetic field;
removing the lysate using the second pipetting mechanism after collecting the beads by means of the magnetic field; and
transferring the first plate with the lysate removed to the section for nucleic acids extraction.

In an embodiment, in step (b), the method comprises:
dispensing 400 to 600 µL of the first wash buffer into the first plate, followed by transferring the first plate to the section for nucleic acid extraction.
shaking the first plate in the section for nucleic acid extraction;
transferring the first plate to the section for applying magnetic field;
removing the first wash buffer using the second pipetting mechanism after collecting the beads by means of the magnetic field; and
transferring the first plate from which the first wash buffer has been removed to the section for nucleic acid extraction

In an embodiment, in step (b), the second wash buffer and/or the third wash buffer are applied in the same manner as the first wash buffer, and the method comprises:
transferring the first plate from which the second wash buffer and/or the third wash buffer have been removed to the section for nucleic acid extraction, followed by incubating the first plate.

In an embodiment, in step (b), the method comprises:
dispensing 50 to 200 µL of the elution buffer into the first plate, followed by shaking the first plate.

In an embodiment, the first plate having 96 wells is a 96 deep-well plate.

In an embodiment, the section for nucleic acid extraction is configured to perform any one of the processes selected from the group consisting of stirring, shaking, ultrasonication, vortexing, thermal treatment, pipetting and combinations thereof.

In an embodiment, the nucleic acid extraction reagent vessel is a 96 deep-well plate or a reservoir plate having a single well.

In an embodiment, the second plate is a 96 well plate.

In an embodiment, the step (c) is performed before, concurrently with, or after step (a).

In an embodiment, the nucleic acid amplification components comprise (i) an oligonucleotide set specific to a target nucleic acid, (ii) DNA polymerases, and optionally reverse transcriptase (RTase) or uracil-DNA glycosylase (UDG), and (iii) dNTP.

In an embodiment, the step (d) is performed by the second pipetting mechanism equipped with the pipette tip having a capacity of 200 to 400 µL.

In an embodiment, the preparation of the nucleic acid extraction and amplification reaction mixture by the method is performed in a time reduced by 30 to 80% compared to the time required when performed in a sample handling system that includes only the first pipetting mechanism.

In another aspect of the invention, the present disclosure provides a sample handling system for preparing reaction mixtures for amplification reaction of nucleic acids in samples, comprising a first pipetting mechanism including 4 to 8 pipetting channels for aspirating and dispensing metered liquids, a second pipetting mechanism including 96 pipetting channels for aspirating and dispensing metered liquids, a section for mounting a first plate having 96 wells, a section for mounting a second plate having 96 wells, a section for nucleic acid extraction, a section for applying magnetic field, a section for mounting sample vessel, a section for mounting a nucleic acid extraction reagent vessel, a section for mounting a nucleic acid amplification component vessel, a section for mounting pipette tips for the pipetting channels of the first pipetting mechanism and a section for mounting pipette tips for the pipetting channels of the second pipetting mechanism, wherein the first pipetting mechanism is configured to move the 4 to 8 pipetting channels independently, and the second pipetting mechanism is configured to move all 96 pipetting channels together, wherein each pipetting channel has a pipette tip mounted or mountable thereon.

In an embodiment, the first pipetting mechanism and the second pipetting mechanism are located separately within the sample handling system.

In an embodiment, the first pipetting mechanism is configured to move 4 to 8 pipetting channels in a direction of X-axis simultaneously, and to move them in directions of Y-axis and Z-axis independently.

In an embodiment, the sample handling system further comprises plate grippers configured to move the first plate and the second plate from one section to another, wherein the plate grippers are mounted on two pipetting channels of the first pipetting mechanism, the two pipetting channels being arranged on opposite sides of the plate respectively such that the plate grippers grip and move the plate.

In an embodiment, the second pipetting mechanism is configured to dispense the nucleic acid extraction reagent into each well of the first plate and dispense the extracted nucleic acid into each well of the second plate.

### [Advantageous Effects]

The features and advantages of the present invention are summarized as follows:
(a) The method of the present disclosure enables faster extraction of nucleic acids and preparation of reaction mixtures for nucleic acid amplification by using a sample handling system that includes a first pipetting mechanism with 4 to 8 independently operable pipetting channels and a second pipetting mechanism with 96 pipetting channels.
(b) The method of the present disclosure simplifies the nucleic acid extraction protocol by using a lysis/binding buffer that does not contain proteinase K and specific wash buffers, thereby reducing the turnaround time while maintaining the reliability of target nucleic acid detection, making it useful in institutions requiring molecular diagnostics for large volumes of samples.
(c) The method of the present disclosure improves the efficiency of molecular diagnostic methods using nucleic acid amplification by reducing the time required for nucleic acid extraction through system optimization, such as using plates preloaded with magnetic beads and/or 96-well plates containing nucleic acid extraction reagents for each step.

### [Specific contents for the implementation of the invention]

In one aspect of this invention, there is provided a method for preparing reaction mixtures for amplifying nucleic acids present in samples using a sample handling system,
wherein the sample handling system comprises a first pipetting mechanism including 4 to 8 pipetting channels for aspirating and dispensing metered liquids, and a second pipetting mechanism including 96 pipetting channels for aspirating and dispensing metered liquids, wherein the first pipetting mechanism is configured to move the 4 to 8 pipetting channels independently, and the second pipetting mechanism is configured to move all 96 pipetting channels together, wherein each pipetting channel has a pipette tip mounted or mountable thereon,
wherein the method comprises:
   (a) dispensing a plurality of samples into each well of a first plate having 96 wells using the first pipetting mechanism;
   (b) extracting nucleic acids from the samples by dispensing a nucleic acid extraction reagent into each well of the first plate using the second pipetting mechanism;
   (c) preparing a nucleic acid amplification reagent by combining nucleic acid amplification components, and then dispensing the nucleic acid amplification reagent into each well of a second plate having 96 wells using the first pipetting mechanism; and
   (d) dispensing nucleic acids extracted in step (b) into each well of the second plate by using the second pipetting mechanism.

### <Sample Handling System>

The term used herein "a sample handling system" refers to an experimental device and/or method for manipulating samples taken from a subject. More specifically, it is a system, having an arrangement of containers such as tubes and plates, configured for processing molecular, cellular, bacterial, or degraded substances in samples in environmental, medical, biological or biochemical laboratory by controlling specific conditions (e.g., temperature, pH) and for transferring, dispensing, and mixing various reagents and samples with extremely small volumes ranging between microliters and milliliters.

Herein, the sample handling system is used to prepare reaction mixtures for nucleic acid amplification reactions in samples. This process is referred to in the art as "set-up" or "preparation" of nucleic acid amplification reactions. The reaction mixtures are prepared through a set-up or preparation process that includes the steps of extracting nucleic acids from the sample and mixing the extracted nucleic acids with nucleic acid amplification reagents.

As used herein, the term "subject" refers to an individual suspected of comprising a target nucleic acid (e.g., a specific pathogen) to be detected using the method of the present disclosure. Examples of the subject include, but are not limited to, mammals such as a dog, a cat, a rodent, a primate, a human or environmental samples such as water, soil, and air.

The term used herein "sample" refers to any cell, tissue, or fluid from a biological source, or any other medium that can advantageously be evaluated according to this invention, including virus, bacteria, tissue, cell, blood, serum, plasma, lymph, milk, urine, faeces, ocular fluid, saliva, semen, brain extracts, spinal cord fluid (SCF), appendix, spleen and tonsillar tissue extracts, amniotic fluid, ascitic fluid and non-biological samples (e.g., food, water, soil). In addition, the sample includes natural-occurring nucleic acid molecules isolated from biological sources and synthetic nucleic acid molecules. Further, the sample may be lysate, extract, or isolated target nucleic acid itself for a specific specimen. The term sample encompasses one harvested from a subject or one that has been subjected to a further process such as extraction, etc. The sample may be used interchangeably with "specimen" herein.

As used herein, the term "nucleic acid," "nucleotide sequence" or "nucleic acid molecule" refers to a single- or double-stranded deoxyribonucleotide or ribonucleotide polymer in which the nucleotides include derivatives of naturally occurring nucleotides, non-naturally occurring nucleotides, or modified nucleotides, which are capable of functioning in the same manner as naturally occurring nucleotides.

The sample handling system applied in the present disclosure comprises a first pipetting mechanism and a second pipetting mechanism. As used herein, the term "pipetting mechanism" refers to a driving element within the sample handling system that can perform pipetting functions for aspirating and/or dispensing precise volumes of a liquid, such as liquid samples, liquid reagents, and/or liquids containing beads, for processing samples, reagents, and/or beads, and driving functions for moving the working position or imparting dynamic movements such as vibration/stirring. The pipetting mechanism comprises the pipetting channel for aspirating and dispensing metered liquids, wherein each pipetting channel has a pipette tip mounted or mountable thereon.

The first pipetting mechanism includes 4 to 8 pipetting channels. The number of pipetting channels may be adjusted by a skilled person considering the available space in the sample handling system and working efficiency. In an embodiment, the first pipetting mechanism includes 5, 6, 7, or 8 pipetting channels. In a specific embodiment, the first pipetting mechanism includes 8 pipetting channels.

According to the present disclosure, the first pipetting mechanism is used when the liquid cannot be processed simultaneously by the second pipetting mechanism including 96 pipetting channels or when multiple replicates of the liquid to be measured, such as 48 or more replicates (for example, 96 replicates), cannot be prepared. For instance, samples are typically collected from a subject in small quantities and stored in a single container, making it challenging to prepare a large number of replicates for simultaneous processing by the second pipetting mechanism. Therefore, these samples can be aspirated and dispensed using the first pipetting mechanism rather than the second pipetting mechanism.

In an embodiment, the first pipetting mechanism is used for aspirating and dispensing samples. In another embodiment, the first pipetting mechanism is used for aspirating and dispensing an internal control (IC). In still another embodiment, the first pipetting mechanism is used for aspirating and dispensing a negative control (NC). In another embodiment, the first pipetting mechanism is used for aspirating and dispensing nucleic acid amplification components and reagents. In still another embodiment, the first pipetting mechanism is used for transferring the first plate or the second plate.

The 4 to 8 pipetting channels included in the first pipetting mechanism can be arranged in various ways within the sample handling system.

In an embodiment, the 4 to 8 pipetting channels included in the first pipetting mechanism are arranged in a row along the Y-axis.

In another embodiment, the 4 to 8 pipetting channels included in the first pipetting mechanism are arranged in two or more rows. For example, when the first pipetting mechanism includes 8 pipetting channels, the pipetting channels can be arranged in a 4 × 2 (4 horizontal, 2 vertical) or 2 × 4 (2 horizontal, 4 vertical) configurations.

Further, the first pipetting mechanism may be configured to move the pipetting channels in directions of X-axis, Y-axis and Z-axis independently. For example, when the first pipetting mechanism includes 8 pipetting channels, the eight pipetting channels may be driven independently for the aspiration and dispensing of reagents and samples, as well as for the movement of plates and tubes.

The term used herein "X-axis," "Y-axis," and "Z-axis" may refer to both directions in which each axis of an orthogonal coordinate system extends when establishing an orthogonal coordinate system at any point within the space of the sample handling system. In an embodiment, a plane parallel to the bottom surface of the sample handling system, that is, the plane on which the plate is placed, may be referred to as the XY plane, and the axis in the direction perpendicular to the bottom surface may be referred to as the Z-axis. Further, a predetermined direction within the XY plane may be referred to as the X-axis, and a direction orthogonal to the X-axis within the XY plane may be referred to as the Y-axis. For example, the horizontal axis within the XY plane may be referred to as the X-axis (e.g., horizontal axis), and the vertical axis orthogonal to the X-axis may be referred to as the Y-axis (e.g., vertical axis). For example, when viewing the sample handling system from the front, the horizontal axis may be designated as the X-axis, the vertical axis in the direction orthogonal to the X-axis and parallel to the bottom surface may be referred to as the Y-axis, and the axis in the direction perpendicular to the bottom surface may be referred to as the Z-axis.

Herein, the X-axis and Y-axis refer to the directions in which the pipetting channels are transferred to the desired section, and the Z-axis refers to the direction in which the pipetting channels enter a specific well within the plate to function as a gripper channel, which will be described later.

In an embodiment, the first pipetting mechanism is configured to move the pipetting channels in the direction of the X-axis simultaneously, and to move them in the directions of the Y-axis and Z-axis independently. Specifically, when the first pipetting mechanism includes 8 pipetting channels, the entire mechanism moves together in the direction of the X-axis, and each pipetting channel can be independently moved to the desired operational position in the directions of the Y-axis and Z-axis. In other words, the first pipetting mechanism is designed to allow individual or partial control of each pipetting channel.

For example, when the first pipetting mechanism includes 8 pipetting channels, 4 pipetting channels can move to a section where tubes containing samples are arranged for dispensing the samples into a plate, and the remaining 4 pipetting channels can move to sections where respective tubes containing nucleic acid amplification components are arranged for preparing a nucleic acid amplification reagent. Further, when the first pipetting mechanism includes 4 to 8 pipetting channels, 2 pipetting channels can be driven to transfer a specific plate with the grippers inserted, and the remaining 2 to 6 pipetting channels do not move and remain at an initial position of the first pipetting mechanism. The term "initial position" refers to the position within the sample handling system where the pipetting mechanism is situated immediately after the sample handling system is turned on/off.

The second pipetting mechanism comprised in the sample handling system includes 96 pipetting channels.

According to the present disclosure, the second pipetting mechanism is used when a large number of liquids can be processed simultaneously or when multiple replicates of the liquid to be measured, such as 48 or more replicates (for example, 96 replicates), can be prepared. For instance, nucleic acid extraction reagents, such as lysis/binding buffer, first wash buffer, second wash buffer, and elution buffer, are commonly used for all samples and thus can be prepared in multiple replicates for simultaneous processing by the second pipetting mechanism. Therefore, these reagents can be aspirated and dispensed quickly and easily by using the second pipetting mechanism rather than the first pipetting mechanism.

In an embodiment, the second pipetting mechanism is used for aspirating and dispensing proteinase K into each of the 96 wells of the plate, with all 96 pipetting channels moving simultaneously. In another embodiment, the second pipetting mechanism is used for aspirating and dispensing lysis/binding buffer, or separately lysis buffer and binding buffer, into each of the 96 wells, with all pipetting channels moving simultaneously. In an embodiment, the second pipetting mechanism is used for aspirating and removing lysate, with all pipetting channels moving simultaneously. In another embodiment, the second pipetting mechanism is used for aspirating and dispensing the first wash buffer into each of the 96 wells, with all pipetting channels moving simultaneously, or for aspirating and removing the first wash buffer in the same manner. In an embodiment, the second pipetting mechanism is used for aspirating and dispensing the second wash buffer into each of the 96 wells, with all pipetting channels moving simultaneously, or for aspirating and removing the second wash buffer simultaneously. In another embodiment, the second pipetting mechanism is used for aspirating and dispensing the elution buffer into each of the 96 wells, with all pipetting channels moving simultaneously, or for aspirating and removing the elution buffer in the same manner. In still another embodiment, the second pipetting mechanism is used for aspirating and dispensing the extracted nucleic acids into each of the 96 wells, with all pipetting channels moving simultaneously.

The 96 pipetting channels included in the second pipetting mechanism can be arranged to fit the size of the plate having 96 wells being used, allowing each pipetting channel to be assigned to each well.

In an embodiment, the 96 pipetting channels included in the second pipetting mechanism are arranged in a configuration of 8 × 12 (8 rows and 12 columns) or 12 × 8 (12 rows and 8 columns).

In an embodiment, the second pipetting mechanism is configured to move 96 pipetting channels in directions of X-axis, Y-axis and Z-axis simultaneously. That is, the 96 pipetting channels included in the second pipetting mechanism may always be positioned in the same section within the sample handling system. For example, the 96 pipetting channels of the second pipetting mechanism may operate such that they move to a section equipped with a nucleic acid extraction reagent, aspirate the reagent simultaneously, and then move to a section with the first plate mounted, where they dispense the reagent simultaneously to dispense a nucleic acid extraction reagent into the first plate containing samples. That is, the 96 pipetting channels of the second pipetting mechanism can perform the same task in the same section.

Meanwhile, the 96 pipetting channels of the second pipetting mechanism do not always aspirate or dispense simultaneously. For example, when there are 48 samples or fewer, only 48 or fewer of the 96 pipetting channels may perform the aspiration and dispensing operations, while the remaining channels may not perform any operations. The number of channels to be activated among the 96 pipetting channels, as well as which channels in the arrangement of the 96 pipetting channels will be activated, can be easily adjusted by those skilled in the art.

In an embodiment, the first pipetting mechanism and the second pipetting mechanism are located separately within the sample handling system. The first pipetting mechanism and the second pipetting mechanism operate as independent units, and the driving of the first pipetting mechanism does not affect the driving of the second pipetting mechanism, nor does the driving of the second pipetting mechanism affect the driving of the first pipetting mechanism. The first pipetting mechanism and the second pipetting mechanism have spatially separated working positions within the sample handling system, and they may be independently operated in a motion path that prevents interference through control elements designed to avoid collisions.

In an embodiment, the first pipetting mechanism is configured to move 4 to 8 pipetting channels in a direction of X-axis simultaneously, and to move them in directions of Y-axis and Z-axis independently. For example, while 8 pipetting channels move simultaneously in the direction of the X-axis, only 2 of the pipetting channels can move in the direction of the Y-axis to a section equipped with grippers, and they can be equipped with grippers as they move in the direction of the Z-axis.

In an embodiment, the second pipetting mechanism is configured to move 96 pipetting channels in the direction of Z-axis independently. For example, the 96 pipetting channels of the second pipetting mechanism can move in the same direction relative to the bottom surface, while also moving independently in a direction perpendicular to the bottom surface.

In an embodiment, the first pipetting mechanism and the second pipetting mechanism can move simultaneously in the directions of the X-axis and Y-axis. The first pipetting mechanism and the second pipetting mechanism are installed as essentially separate elements with a predetermined interval within the sample handling system, but they can move simultaneously in the direction of the X-axis and Y-axis to perform their respective operations. In this case, the mechanism performing the desired work can move its pipetting channels in the direction of Z-axis to aspirate or dispense liquids, while the mechanism not performing any operation may have its pipetting channels stationary in the Z-axis direction.

The present invention maximizes the efficiency of molecular diagnostics using nucleic acid amplification reactions by employing the sample handling system that utilizes both types of pipetting mechanisms.

Conventional sample handling systems generally comprise a pipetting mechanism with 8 or fewer pipetting channels, wherein one or more channels are capable of independent movement, and all pipetting operations (from dispensing samples to removing reagents) are carried out using this mechanism. Even when performing the same operations on a 96-well plate, the mechanism had to be operated repeatedly for each well, which led to considerable unnecessary time consumption, and there was a concern that detection errors would occur due to variations in the reagent dispensing and removal times for each sample.

To address these issues, the present inventors applied the second pipetting mechanism for performing identical operations on the first plate and for dispensing the extracted nucleic acids into the second plate, thereby significantly reducing the assay time. For other tasks, the first pipetting mechanism was used to perform more detailed operations, resulting in a faster, more accurate, and improved molecular diagnostic method.

In addition to the first pipetting mechanism and the second pipetting mechanism described above, the sample handling system according to the present disclosure may further comprise various components necessary for preparing reaction mixtures for nucleic acid amplification reactions. The sample handling system may have compartments or spaces for mounting these various components.

In an embodiment, the sample handling system comprises a section for mounting the first plate, a section for mounting the second plate, a section for nucleic acid extraction, and a section for applying a magnetic field.

The first plate is a container in which all steps for extracting nucleic acids from samples are performed, and when the nucleic acid extraction process is completed, the extracted nucleic acids are contained therein. The second plate is a container in which the nucleic acids from the first plate are mixed with nucleic acid amplification reagents. In the second plate, nucleic acid amplification components and the extracted nucleic acids are combined. That is, the setup for the nucleic acid amplification reaction is completed in the second plate before the reaction is initiated, and the second plate can be applied to a nucleic acid amplification system to start the reaction. The section for mounting the first plate and/or the section for mounting the second plate may be present in one or more instances.

The sample handling system may comprise a section for nucleic acid extraction and a section for applying magnetic field, allowing various processes necessary for extracting nucleic acids to be performed in the first plate. Specifically, the section for nucleic acid extraction may be a thermal/stirring deck, and the section for applying magnetic field may be a magnetic field or magnetic deck.

For example, the first plate containing beads, samples, and lysis and binding buffers can be heat treated and/or stirred in the section for nucleic acid extraction according to the characteristics of the reagents. Thereafter, the first plate is transferred to the section for applying magnetic field (a section equipped with magnets) to collect the beads bound with nucleic acids via magnetic force, allowing the lysate to be removed using the pipetting channels of the pipetting mechanism.

In other words, the first plate and the second plate can be transferred to various sections based on the type of step-by-step operations, rather than being fixed at a single location within the workstation of the sample handling system.

In an embodiment, the sample handling system further comprises a section for mounting sample vessel, a section for mounting a nucleic acid extraction reagent vessel and a section for mounting a nucleic acid amplification component vessel.

The section for mounting sample vessels may be implemented in the same or different form as the section for mounting other vessels.

For example, each of the samples may be provided in a form accommodated in each well of a 96-well plate, so the section for mounting the sample vessels may be a section for mounting plates. In this case, either the first pipetting mechanism or the second pipetting mechanism can be used to dispense the samples into the first plate.

Further, the section may serve as a carrier capable of arranging each sample tube itself, which may take the form of i) up to 96 sample tubes, ii) up to 95 sample tubes and an internal control tube, or iii) up to 94 sample tubes, an internal control tube, and a negative control tube inserted.

The section for mounting the nucleic acid extraction reagent vessels refers to a section where all reagent vessels used in the extraction can be mounted, depending on the extraction method. In an embodiment, the section for mounting proteinase K vessel, the section for mounting lysis buffer vessel, the section for mounting binding buffer vessel, the section for mounting first wash buffer vessel, the section for mounting second wash buffer vessel, the section for mounting third wash buffer vessel, and the section for mounting elution buffer vessel may be included. In an embodiment, the section for mounting lysis/binding buffer vessel, the section for mounting first wash buffer vessel, the section for mounting second wash buffer vessel, and the section for mounting elution buffer vessel may be included. The sections may be positioned adjacent to each other or separately. Further, as reagent vessels required for each step are mounted at the same location in a time-staggered manner, sections equipped with specific reagent vessels may overlap.

The section for mounting the nucleic acid amplification component vessel may include a section for mounting a vessel containing a set of oligonucleotides specific to the target nucleic acid, a section for mounting a vessel containing enzymes such as DNA polymerase, and optionally reverse transcriptase (RTase) or uracil-DNA glycosylase (UDG), and a section for mounting a vessel containing dNTPs. These sections may be positioned adjacent to each other or separately.

In an embodiment, the sample handling system further comprises the section for mounting pipette tips for the pipetting channels of the first pipetting mechanism, and the section for mounting pipette tips for the pipetting channels of the second pipetting mechanism. Due to the different operating methods of the first and second pipetting mechanisms, the sections equipped with pipettes used for each pipetting mechanism may differ.

In an embodiment, the sections for mounting pipette tips each comprise a section equipped with pipette tips having a capacity of 200 to 400 µL, and a section equipped with pipette tips having a capacity of 900 to 1,100 µL. Pipettes mounted for dispensing/aspirating reagents for nucleic acid extraction, mixing nucleic acid amplification components, aspirating/dispensing extracted nucleic acids, and other operations can be selected with appropriate capacities depending on the applicable step. For example, each of the sections for mounting pipette tips for the pipetting channels of the first and second pipetting mechanisms may separately comprise a section equipped with pipette tips having capacity of 200, 250, 300, 350, or 400 µL, and a section equipped with pipette tips having capacity of 900, 950, 1,000, 1,050, or 1,100 µL. This configuration allows efficient setup of the nucleic acid amplification reaction by enabling the use of suitable pipettes for each step.

In an embodiment, the sample handling system further comprises plate grippers configured to move the first plate and the second plate from one section to another.

The first plate and second plate are containers that provide a space where various components are mixed/accommodated. Therefore, these plates need to be transferred to sections where the necessary processing can be performed according to each step performed for nucleic acid amplification reaction setup. In this regard, the plate grippers can grab the plate to be transferred and transfer it to the section where the next step will take place.

In an embodiment, the plate grippers are mounted on two pipetting channels of the first pipetting mechanism, the two pipetting channels being arranged on opposite sides of the plate respectively such that the plate grippers grip and move the plate.

To this end, the pipetting channel of the first pipetting mechanism may be equipped with plate grippers thereon instead of a pipetting tip. Specifically, unlike the second pipetting mechanism, the first pipetting mechanism can move the pipetting channels individually in the X-axis, Y-axis, and Z-axis directions. This individual operating mode is also necessary for aspiration/dispensing liquids but is essential when performing specific plate transfer tasks.

When the plate grippers are mounted on the two pipetting channels of the first pipetting mechanism, the pipetting channels that equipped with the plate grippers (the gripper channels) are positioned on opposite sides of the plate to be transferred. For example, in the case of a 96-well plate, the two gripper channels can be positioned on each side of an 8-well length, facing each other. The remaining 2 to 6 pipetting channels do not perform any special tasks within the first pipetting mechanism. The plate grippers may be present as 2 to 4 or 2 within the sample handling system. While the pipette tips are changed when the type of liquid being aspirated/dispensed changes, the plate grippers can be reused by remaining positioned in a specific section.

Specifically, when the gripper channels are needed for plate transfer, the first pipetting mechanism moves to a section where the plate grippers are located and implements the gripper channel by mounting the plate grippers onto two specific pipetting channels. Then, the first pipetting mechanism, equipped with the gripper channel moves to a section where a target plate exists, grips the plate with the gripper channels and transfers it to the desired section. Once the transfer of the target plate is complete, the first pipetting mechanism returns to the section where the plate grippers were originally located and releases the plate grippers from the pipetting channels.

### <Preparation of Reaction Mixtures>

### Step (a): Dispensing of Samples Using the First Pipetting Mechanism

According to the present disclosure, in step (a), a plurality of samples is dispensed into each well of the first plate having 96 wells using the first pipetting mechanism.

In an embodiment, the sample may be obtained from a subject, particularly a mammal, more particularly a human, and may be, for example, a swab, saliva, sputum, aspiration, bronchoalveolar lavage (BAL), gargle, or blood, but is not limited thereto.

In certain embodiments, the sample derived from the subject is a swab, saliva, or a mixture thereof.

In an embodiment, the swab is nasopharyngeal swab, nostril swab, oropharyngeal swab, oral swab, saliva swab, genital swab, rectal swab or a mixture of two or more thereof.

In an embodiment, the target nucleic acid may be a nucleic acid of a human, animal, plant, or microorganism. The microorganism may be fungi, protozoa, bacteria, viruses, or algae.

In an embodiment, the target nucleic acid may be a viral nucleic acid, and specifically, the target nucleic acid may be an RNA viral nucleic acid.

In an embodiment, the target nucleic acid may be a respiratory virus nucleic acid. For example, the target nucleic acid may be an influenza virus nucleic acid, a respiratory syncytial virus (RSV) nucleic acid, an adenovirus nucleic acid, an enterovirus nucleic acid, a parainfluenza virus nucleic acid, a metapneumovirus (MPV) nucleic acid, a bocavirus nucleic acid, a rhinovirus nucleic acid, and/or a coronavirus nucleic acid, but is not limited thereto.

In an embodiment, the target nucleic acid may be a nucleic acid of SARS-CoV-2.

Further, the sample may be non-biological samples such as soil, water, food.

The samples can be prepared as multiple reaction mixtures in a short time using the sample handling system according to the present disclosure. The number of samples that can be prepared using the sample handling system according to the present disclosure can be 96 or fewer and can be adjusted based on the number of controls used. For example, the number of samples that can be prepared using the sample handling system according to the present disclosure can be 94, with the remaining 2 being Internal controls (positive control and negative control).

The sample derived from the subject may be obtained using various sample collection devices. The term "sample collection device" is used to encompass the means, tools, and other necessary instruments for collecting and retaining the collected sample.

In an embodiment, the sample collection device comprises a vessel capable of holding a sample.

In another embodiment, the sample collection device comprises a transport medium. The transport medium refers to a preservation transport medium capable of maintaining sample integrity, such as cell integrity of a virus or bacteria.

Examples of such transport medium include commercially available products such as Copan Universal Transport Medium (UTM), Viral Transport Medium (VTM), or Clinical Virus Transport Medium (CTM) from Noble Biosciences, Inc., but is not limited thereto.

In an alternative embodiment, the preservation transport medium may be a saline-based solution or a balanced salt solution-based medium. For example, the saline-based solution may be phosphate buffered saline (PBS) or normal saline.

In an embodiment, the transport medium does not include a material for lysis. In the present disclosure, the term "material for lysis" refers to components used to lyse a specimen (*i.e.*, cells) by chemical cell lysis methods, which may include, for example, chaotropic agents.

In an embodiment, the transport medium may be a liquid medium.

According to the present disclosure, an internal control (IC) may be dispensed before dispensing the sample. In an embodiment, the internal control is dispensed using the first pipetting mechanism. The internal control is included to verify the reliability of the nucleic acid amplification reaction result and is not limited to materials used in the relevant technical field. For example, ACTB (GenBank ID: NM_001101.2) or MS2 phage (ATCC, Cat No. 15597) may be used as the internal control.

In an embodiment, in step (a), the internal control is dispensed using the first pipetting mechanism equipped with a pipette tip having a capacity of 200 to 400 µL. The samples are dispensed using the first pipetting mechanism equipped with a pipette tip having a capacity of 900 to 1,100 µL, preferably 1,000 µL, while the internal control is dispensed using the first pipetting mechanism equipped with a pipette tip having a capacity of 200 to 400 µL, preferably 300 µL.

In an embodiment, the first plate having 96 wells is a 96 deep-well plate. Among multi-well plates, the deep-well plates are designed to accommodate a larger volume of samples than a general well plates. Since the first plate serves as a reaction vessel where a mixture of beads, samples, and various buffers is performed, using a deep-well plate with deeper wells can minimize cross-contamination. Furthermore, the material of the 96 deep-well plate may be polypropylene (PP), which does not undergo deformation due to heat or chemical reactions, ensuring stable nucleic acid extraction processes can be performed in the first plate.

The 96 deep-well plate may be a commercially available plate, with examples including plates from Bio-Rad, Thermo Fisher, and Sigma Aldrich.

In an embodiment, the first plate is preloaded with magnetic beads that bind to nucleic acids. The first plate may be in a pre-filled state with magnetic beads for use in nucleic acid extraction reactions. The magnetic beads can be suspended in RNase-free water before being added to the first plate.

### Step (b): Dispensing Nucleic Acid Extraction Reagents Using the Second Pipetting Mechanism

In step (b) of the present disclosure, nucleic acids are extracted from the samples by dispensing nucleic acid extraction reagents into each well of the first plate using the second pipetting mechanism.

The term "nucleic acid extraction reagent" as used herein refers to a collection of multiple reagents used specifically for extracting nucleic acids from samples. The nucleic acid extraction reagents may be filled in separate containers.

In general, the extraction of nucleic acids requires a series of processes: lysis, binding, wash, purification, and elution. The collected samples are incubated in a lysis buffer to destroy cell walls and membranes, thereby exposing the nucleic acids, which are then bound to specific structures such as membranes or beads. Afterwards, buffers are used at each step to separate and wash the pure nucleic acids from the structures. Therefore, conventional nucleic acid extraction methods require at least one reagent for each step, and three types of wash buffers are generally used.

As such, the nucleic acid extraction consists of several stages and a variety of operational steps, including reagent aspiration, dispensing, and redistribution, which lead to extended processing times that considerably impact the turnaround time (TAT) for nucleic acid amplification reaction setups. Therefore, the present disclosure provides a sample handling system that offers a configuration allowing for a more streamlined and efficient nucleic acid extraction process, thereby significantly reducing the time required for preparing samples for nucleic acid amplification.

The nucleic acid extraction step (b) of the present invention can be broadly categorized into the step of binding nucleic acids to magnetic beads after sample lysis, the step of washing while the nucleic acids are bound to the magnetic beads, and the step of dispensing the elution buffer after removing the wash buffer and incubating. The step of binding nucleic acids to magnetic beads after sample lysis has some different operations depending on whether the lysis buffer and binding buffer are separate or integrated. Hereinafter, specific embodiments are described according to the combinations of nucleic acid extraction reagents.

In an embodiment, the nucleic acid extraction reagents include a lysis/binding buffer, a first wash buffer, a second wash buffer, and an elution buffer. The present invention utilizes a functional buffer with dual activities that aids in binding nucleic acids to magnetic beads while lysing the cells to expose the nucleic acids, thereby reducing the lysis and binding steps into a single stage. Additionally, the composition/combination of the entire reagents was configured so that only two types of wash buffers can achieve nucleic acid yield levels comparable to those of conventional methods.

In an embodiment, in step (a), the first plate is preloaded with magnetic beads that bind to nucleic acids, and the samples can be dispensed after mounting the first plate in the section for nucleic acid extraction.

Generally, nucleic acid extraction methods using magnetic beads also take time for the process of dispensing beads prepared in separate tubes. In contrast, the present invention reduces the processing time by applying a 96-well plate, more preferably a 96 deep-well plate, that is preloaded with magnetic beads. Furthermore, when dispensing magnetic beads within the sample handling system, some magnetic beads may be minimally retained in the pipetting channels or the device itself, potentially causing mechanical errors or some experimental inaccuracies. Therefore, the present invention addresses this issue by using a first plate that is preloaded with magnetic beads.

In an embodiment, the first plate may undergo one of the processes selected from a group consisting of stirring, shaking, ultrasonication, vortexing, thermal treatment, pipetting and combinations thereof, to minimize the agglomeration of the preloaded magnetic beads. When using a first plate that is preloaded with magnetic beads, some beads may aggregate due to temperature or humidity issues during storage. Such agglomerated beads can lead to varying amounts being dispensed from each well, causing discrepancies in the nucleic acid extraction efficiency from the samples. Therefore, by mounting the first plate in a section of the sample handling system that allows for such suspension methods, physical or thermal stress can be applied to the plate to disperse or prevent the agglomeration of the magnetic beads.

According to a specific embodiment, after being collected from the subject, the sample is placed in a sample vessel and mounted in a sample vessel section of the sample handling system of the present invention. Thereafter, the sample handling system of the present invention dispenses the sample from the sample vessel into each well of the first plate using the first pipetting mechanism. Herein, the first plate is a 96-deep well plate, and magnetic beads for nucleic acid extraction are preloaded. As a result, the sample and the magnetic beads are mixed in each well.

In an embodiment, in step (b), the method may comprise transferring the first plate to the section for applying magnetic field after dispensing 300 to 500 µL of the lysis/binding buffer into the first plate, removing the lysate using the second pipetting mechanism after collecting the beads by means of the magnetic field, and transferring the first plate with the lysate removed to the section for nucleic acid extraction.

The first plate, with the sample and magnetic beads mixed, can receive 300 to 500 µL, preferably 400 µL, of the lysis/binding buffer to perform cell lysis and binding of nucleic acids to the beads. Before performing this, the second pipetting mechanism moves to a section equipped with 900 to 1,100 µL pipettes, installs the required pipettes on the pipetting channels, and then proceeds to the location where a plate containing lysis/binding buffer is positioned to simultaneously aspirate the buffer into each pipette. Afterwards, the second pipetting mechanism moves to the location where the first plate is mounted, dispenses the lysis/binding buffer simultaneously, and incubates for a predetermined period of time. Subsequently, the first pipetting mechanism moves to a section equipped with grippers, installs the gripper on at least two pipetting channels, and then moves to the location where the first plate is mounted to grip the first plate and transfer it to the magnetic section.

The section for applying magnetic field may have magnetic beads concentrated rather than dispersed in the solution due to the magnetic properties of its bottom surface. Thus, while the beads are collected using magnetism, the second pipetting mechanism can simultaneously remove the lysate from each well. Thereafter, the first pipetting mechanism, equipped with grippers, moves the first plate, from which the lysate has been removed, to the section for nucleic acid extraction.

In an embodiment, the nucleic acid extraction reagent comprises proteinase K, a lysis buffer, a binding buffer, a first wash buffer, a second wash buffer, a third wash buffer and an elution buffer.

This is a combination of extraction reagents that can perform the most common nucleic acid extractions, where various known reagents can be appropriately applied at each step according to their functions.

In one embodiment, before dispensing the sample in step (a), the first plate is mounted in the section for nucleic acid extraction, and proteinase K can be dispensed in an amount of 3 to 20 µL, 5 to 15 µL, 7 to 13 µL, or 10 µL.

Proteinase K is a proteolytic enzyme used to efficiently and purely isolate target nucleic acids from samples. For example, viral RNA genomes are bound to nucleocapsid proteins, and this enzyme effectively breaks them down, allowing the separation of RNA genomes while inactivating enzymes (proteins) such as DNases and RNases that degrade DNA and RNA present in the sample, thereby enhancing detection efficiency.

However, Proteinase K is sensitive to water and heat, making it challenging to handle and expensive, leading to ongoing research and development for extraction processes that can omit its use. In this regard, the present invention describes a sample handling system that applies a nucleic acid extraction process without Proteinase K in another embodiment. In one embodiment, Proteinase K can be applied and can be dispensed first into an empty first plate.

In one embodiment, before dispensing the sample in step (a), the first plate is mounted in the section for nucleic acid extraction, and proteinase K can be dispensed into each well in an amount of 3 to 20 µL, 5 to 15 µL, 7 to 13 µL, or 10 µL.

In an embodiment, Proteinase K can be dispensed using a second pipetting mechanism equipped with pipette tips having capacity of 200 to 400 µL, 250 to 350 µL, or 300 µL. Using the second pipetting mechanism with the number of pipettes corresponding to the number of samples from which nucleic acids are to be extracted, Proteinase K can be dispensed into up to 96 wells simultaneously.

Proteinase K is a proteolytic enzyme used to efficiently and purely isolate target nucleic acids from samples. For example, viral RNA genomes are bound to nucleocapsid proteins, and this enzyme can effectively degrade these proteins to isolate only the RNA genome, thereby inactivating enzymes (proteins) such as DNases or RNases that may degrade the nucleic acids present in the sample, enhancing detection efficiency. However, due to its sensitivity to moisture and heat, as well as its high cost, research and development for extraction processes that can omit its use are ongoing.

An embodiment of the present invention minimizes the time required to dispense this reagent while applying Proteinase K, thereby reducing the time necessary for setting up nucleic acid amplification reactions.

In an embodiment, in step (b), the method may comprise dispensing 100 to 200 µL of lysis buffer into the first plate, dispensing 3 to 20 µL of magnetic beads into the first plate, dispensing 500 to 700 µL of binding buffer, followed by transferring the plate to the section for applying magnetic field, removing the lysate using the second pipetting mechanism after collecting the beads by means of the magnetic field and transferring the first plate with the lysate removed to the section for nucleic acids extraction.

The above method is a case where a separate extraction reagent is applied in which a lysis buffer and a binding buffer are configured separately. After sample and/or IC dispensing, the lysis buffer, magnetic beads, and binding buffer are applied separately in a sequential order.

The lysis buffer may be dispensed in amounts ranging from 100 to 200 µL, 120 to 180 µL, or approximately 150 µL. In this case, the lysis buffer can be simultaneously dispensed into each well of the first plate from the lysis buffer plate using the second pipetting mechanism.

In an embodiment, the sample handling system further comprises a section for mounting a magnetic bead vessel. If a plate pre-filled with magnetic beads is not used, magnetic beads can be dispensed from the magnetic bead vessel mounted in the section for mounting a magnetic bead vessel into each well of the first plate, which has been dispensed with the sample and lysis buffer, using the first pipetting mechanism in amounts ranging from 3 to 20 µL, 5 to 15 µL, or approximately 10 µL.

The binding buffer can be dispensed in amounts ranging from 500 to 700 µL, 550 to 650 µL, or approximately 600 µL. At this time, the binding buffer can be simultaneously dispensed into each well of the first plate from the binding buffer plate using the second pipetting mechanism.

Subsequently, the first pipetting mechanism moves to a section equipped with grippers, installs the grippers to at least two pipetting channels, and then moves to the location where the first plate is mounted to grip the first plate and transfer it to the section for applying magnetic field. While the beads are collected using magnetism, the second pipetting mechanism can simultaneously remove the lysate from each well. Thereafter, the first pipetting mechanism equipped with grippers transfers the first plate with the lysate removed to the section for nucleic acid extraction.

The lysis buffer, binding buffer, and lysis/binding buffer may include various known substances capable of lysing samples, such as chaotropic agents, chelating agents, buffers, and surfactants. Meanwhile, the lysis/binding buffer does not include Proteinase K.

The chaotropic agent may include guanidine thiocyanate, guanidine hydrochloride, guanidine isothiocyanate, potassium thiocyanate and combinations thereof, but is not limited to thereto. The chelating agent may include EDTA, NTA, DCTA, DTPA, EGTA, and combinations thereof, but is not limited to thereto. Additionally, the buffer may include sodium phosphate buffer, potassium phosphate buffer, Tris-HCl buffer, or Tricine buffer, but is not limited to thereto.

Additionally, the lysis/binding buffer according to an embodiment of the present invention may include two types of surfactants. Specifically, the lysis/binding buffer may contain both a nonionic surfactant and an ionic surfactant. For example, the lysis/binding buffer may comprise the nonionic surfactant and ionic surfactant in a concentration ratio of 20:1 to 5:1, 18:1 to 7:1, 15:1 to 9:1, or 13:1 to 10:1.

The ionic surfactant may be sodium dodecyl sulfate (SDS) or N-lauroyl sarcosine. Further, the nonionic surfactant may comprise polyethylene glycol-based nonionic surfactants, alcohol-based nonionic surfactants, Triton X-100, or Tween 20. By comprising these two types of surfactants, the lysis/binding buffer can effectively dissolve samples to a degree that makes it easy to extract nucleic acids without including proteinase K, and furthermore, the two types of washing buffers can effectively remove contaminants other than nucleic acids.

In an embodiment, in step (b), the method may comprise dispensing 400 to 600 µL of the first washing buffer, followed by transferring the first plate to the section for nucleic acid extraction, shaking the first plate in the section for nucleic acid extraction; transferring the first plate to the section for applying magnetic field, removing the first wash buffer using the second pipetting mechanism after collecting the beads by means of the magnetic field, and transferring the first plate, from which the first wash buffer has been removed, back to the section for nucleic acid extraction.

The wash buffer may include various commercially available types and can be dispensed in an amount of 400 to 600 µL, 420 to 580 µL, 450 to 550 µL, or about 500 µL. All wash buffers can be aspirated from the wash buffer plate and dispensed simultaneously into the first plate using the second pipetting mechanism. Subsequently, the first pipetting mechanism may move to the section equipped with grippers, install the grippers on at least two pipetting channels, and then transfer the first plate to the section for nucleic acid extraction. The section for nucleic acid extraction operates to effectively wash the nucleic acid-bound beads through shaking. Thereafter, the first pipetting mechanism may transfer the first plate to the section for applying magnetic field, and with the magnetic beads collected by magnetism, the second pipetting mechanism removes the wash buffer from the first plate. The first plate, with the wash buffer removed, is then transferred back to the section for nucleic acid extraction by the first pipetting mechanism. All of these processes may be performed in the same operation for all of the first wash buffer, the second wash buffer, and the third wash buffer.

Accordingly, the second wash buffer and/or the third wash buffer may be applied in the same manner as the first wash buffer, and the method may comprise transferring the first plate from which the second wash buffer and/or the third wash buffer has been removed to the section for nucleic acid extraction for incubation. After the final wash buffer treatment, incubation can increase nucleic acid yield during the subsequent application of the elution buffer.

In an embodiment, the first wash buffer comprises a salt containing guanidinium ions. The first wash buffer may include guanidinium salts such as guanidinium thiocyanate, guanidinium hydrochloride, or guanidinium isocyanate, but is not limited thereto.

Further, the first wash buffer may include surfactants, chelating agents, buffers, etc., with non-limiting examples of each component as described above.

In an embodiment, the second wash buffer does not contain water treated with DEPC (Diethyl pyrocarbonate). Specifically, the second wash buffer may include ethanol at a concentration of 70% to 90%, 72% to 88%, 74% to 86%, 78% to 84%, or 80% to 82%, and does not include DEPC. Although water treated with DEPC is often used to obtain pure RNA because it interferes with RNase activity, the presence of DEPC in nucleic acid amplification reagents during nucleic acid amplification reactions, particularly in RT-PCR, may inhibit the amplification reaction. Therefore, the second wash buffer of the present invention is formulated with an optimal ethanol concentration that effectively removes contaminants before nucleic acid elution without using DEPC-treated water.

Next, the method may include dispensing the lysis buffer simultaneously into each well using the second pipetting mechanism in an amount of 50 to 200 µL, 70 to 130 µL, or approximately 100 µL, followed by shaking the first plate. In an embodiment, the elution buffer may be ultra-pure water (UPW) that is nuclease-free but is not limited to thereto.

In an embodiment, the nucleic acid extraction reagents are preloaded into their respective nucleic acid extraction reagent containers. Depending on the extraction reagent combination, the lysis buffer, binding buffer, lysis/binding buffer, wash buffer, and elution buffer are filled in separate containers. In this regard, the nucleic acid extraction reagent containers may be 96 deep-well plates or reservoir plates with a single well. Each reagent may be contained in a plate that holds a sufficient volume to perform multiple nucleic acid extraction processes.

Nucleic acid extraction kits in the art generally provide each reagent in the form of tubes or cartridges, and these tubes or cartridges are placed in specific sections of the sample handling system and dispensed using a predetermined number of pipetting channels, usually fewer than 10. This configuration resulted in a significantly longer time required for the aspiration, dispensing, and removal of reagents at each step of the nucleic acid extraction process compared to the time taken for the extraction reaction itself.

In response, the present inventors have utilized the 96 pipetting channels of the second pipetting mechanism to simultaneously dispense nucleic acid extraction reagents, thereby drastically shortening the reagent dispensing time for each step.

According to a specific embodiment, the lysis/binding buffer is dispensed into each well of the first plate containing the sample and beads using the second pipetting mechanism, where the exposed nucleic acids bind to the magnetic beads. The first plate is then transferred to a section equipped with a magnet, where the magnetic beads bound to the nucleic acids are collected, and the lysate is aspirated and removed. Next, the first wash buffer is dispensed into each well of the first plate containing the magnetic beads bound to the nucleic acids using the second pipetting mechanism. Subsequently, the first plate is moved away from the section equipped with the magnet to wash the nucleic acids, and then transferred back to the section with the magnet, where the magnetic beads bound to the nucleic acids are collected, and the supernatant is aspirated to remove the first wash buffer. After that, the second wash buffer is dispensed into each well of the first plate containing the magnetic beads bound to the nucleic acids using the second pipetting mechanism. The first plate is then moved away from the section equipped with the magnet to wash the nucleic acids, and then transferred back to the section with the magnet, where the magnetic beads bound to the nucleic acids are collected, and the supernatant is aspirated to remove the second wash buffer. Subsequently, the first plate is dried, and the elution buffer is dispensed into each well of the first plate containing the magnetic beads bound to the nucleic acids using the second pipetting mechanism. The plate is then transferred back to the section equipped with the magnet, where the magnetic beads bound to the nucleic acids are collected, and the supernatant is aspirated to collect the eluted nucleic acids. The movement of the plate is performed entirely using the first pipetting mechanism and the plate grippers.

As described above, the method of the present invention utilizes a first plate preloaded with magnetic beads, a plate preloaded with lysis/binding buffer, a plate preloaded with the first wash buffer, a plate preloaded with the second wash buffer, and a plate preloaded with elution buffer. The use of these preloaded reagent plates enables the application of the second pipetting mechanism, allowing for a reduction in the overall testing time.

In another embodiment, a general lysis buffer and binding buffer may be used instead of the lysis/binding buffer. In this case, the entire process of aspiration, dispensing, and removal of the lysate for the lysis/binding buffer can be performed in two steps: i) removal of the lysate after the aspiration/dispensing of the lysis buffer and ii) removal of the supernatant after the aspiration/dispensing of the binding buffer. Additionally, in place of the specific first wash buffer and second wash buffer of the present invention, general first wash buffer, second wash buffer, and third wash buffer can be used. In this case, after the removal of the supernatant following the aspiration/dispensing of the second wash buffer, a step of removing the supernatant after the aspiration/dispensing of the third wash buffer may be added.

As described above, even when the lysis/binding buffer, first wash buffer, and second wash buffer according to one embodiment of the present invention are not applied, the nucleic acid extraction process using the sample handling system of the present invention can significantly reduce the time required for nucleic acid extraction compared to the prior art. This is because the application of the first pipetting mechanism and the second pipetting mechanism simultaneously can shorten the individual time required for each buffer.

The nucleic acids extracted in step (b) may be derived from a human, an animal, a plant, or a microorganism. The microorganism may include a fungus, a protozoan, a bacterium, a virus, an alga, and others.

According to one embodiment, the nucleic acid may be a nucleic acid of a virus, and specifically, the nucleic acid may be a nucleic acid of an RNA virus.

According to one embodiment, the nucleic acid may be a nucleic acid of a respiratory virus. For example, the nucleic acid may comprise a nucleic acid of influenza virus, a nucleic acid of a respiratory syncytial virus (RSV), a nucleic acid of an adenovirus, a nucleic acid of an enterovirus, a nucleic acid of a parainfluenza virus, a nucleic acid of a metapneumovirus (MPV), a nucleic acid of a bocavirus, a nucleic acid of a rhinovirus, and/or a nucleic acid of a coronavirus, etc.

According to one embodiment, the nucleic acid may be a nucleic acid of severe acute respiratory syndrome coronavirus 2 (SARS-CoV-2).

According to one embodiment, the nucleic acid of SARS-CoV-2 may be selected from the group consisting of the E gene, N gene, RdRP gene, S gene, and a combination thereof

### Step (c): Preparing and Dispensing Nucleic Acid Amplification Reagents Using the First Pipetting Mechanism

In this step, the components for nucleic acid amplification are mixed using the first pipetting mechanism to prepare the nucleic acid amplification reagent, which is then dispensed into each well of the second plate having 96 wells.

In an embodiment, the components for nucleic acid amplification may be mixed in tubes, cartridges, reservoir plates, or each well of a 96-well plate, but are not limited to these options. For example, when the dispensing of each component for nucleic acid amplification using the first pipetting mechanism is performed into a 96-well plate or reservoir plate, the mixed nucleic acid amplification reagent can be dispensed at once into each well of the second plate using the second pipetting mechanism. Additionally, if the dispensing of each component for nucleic acid amplification using the first pipetting mechanism is performed in a container in the form of a tube with a predetermined volume, the mixed nucleic acid amplification reagent can be dispensed into each well of the second plate using the first pipetting mechanism.

In an embodiment, the step (c) may be performed before, concurrently with, or after step (a). The components for nucleic acid amplification may be mixed to prepare the nucleic acid amplification reagent in the second plate before dispensing the biological sample into the first plate. Alternatively, the nucleic acid amplification reagent may be prepared in a separate second plate while dispensing the biological sample into the first plate. Additionally, step (c) may be performed before or after step (b) following the dispensing of the biological sample into the first plate.

In an embodiment, the nucleic acid amplification components comprise (i) an oligonucleotide set specific to a target nucleic acid, (ii) DNA polymerases, and optionally reverse transcriptase (RTase) or uracil-DNA glycosylase (UDG), and (iii) dNTP.

In an embodiment, the oligonucleotide sets specific to the target nucleic acid comprises oligonucleotides for amplifying and/or detecting the target nucleic acid (*e.g.*, primers or probes).

In a specific embodiment, the oligonucleotides specific to the target nucleic acid are primers, probes, or a combination thereof.

The term "primer," as used herein, refers to an oligonucleotide which is capable of acting as a point of initiation of synthesis when placed under such a condition that induces the synthesis of a primer extension product complementary to a target nucleic acid (template), that is, in the presence of nucleotides and an agent for polymerization, such as DNA polymerase, and at suitable temperatures and pH. The primer must be long enough to prime the synthesis of extension products in the presence of the agent for polymerization. The exact length of the primers may vary depending on many factors including, for example, temperatures, field of application, and primer sources.

In an embodiment of the present disclosure, the oligonucleotide may have a length of 5 to 1,000 bp, 5 to 900 bp, 5 to 800 bp, 5 to 700 bp, 5 to 600 bp, 5 to 500 bp, 5 to 400 bp, 5 to 300 bp, 5 to 200 bp, 5 to 150 bp, 5 to 100 bp, 5 to 90 bp, 5 to 80 bp, 5 to 70 bp, 5 to 60 bp, 5 to 50 bp, 5 to 40 bp, 5 to 30 bp, 5 to 20 bp, 5 to 10 bp, 10 to 1,000 bp, 10 to 900 bp, 10 to 800 bp, 10 to 700 bp, 10 to 600 bp, 10 to 500 bp, 10 to 400 bp, 10 to 300 bp, 10 to 200 bp, 10 to 150 bp, 10 to 100 bp, 10 to 90 bp, 10 to 80 bp, 10 to 70 bp, 10 to 60 bp, 10 to 50 bp, 10 to 40 bp, 10 to 30 bp, 10 to 20 bp, 15 to 1,000 bp, 15 to 900 bp, 15 to 800 bp, 15 to 700 bp, 15 to 600 bp, 15 to 500 bp, 15 to 400 bp, 15 to 300 bp, 15 to 200 bp, 15 to 150 bp, 15 to 100 bp, 15 to 90 bp, 15 to 80 bp, 15 to 70 bp, 15 to 60 bp, 15 to 50 bp, 15 to 40 bp, or 15 to 30 bp, but with no limitations thereto.

As used herein, the term "probe" means a single-stranded nucleic acid molecule including a portion or portions that are substantially complementary to a target nucleic acid sequence. According to an embodiment of the present disclosure, the 3'-end of the probe may be "block" to prohibit its extension. The blocking may be achieved in accordance with conventional methods. For instance, a chemical moiety such as biotin, a label, a phosphate group, an alkyl group, a non-nucleotide linker, phosphorothioate, or alkane-diol may be linked to the 3'-hydroxyl group of the last nucleotide to block the extension therefrom. Alternatively, the blocking may be carried out by removing the 3'-hydroxyl group of the last nucleotide or by using a nucleotide lacking 3'-hydroxyl group, such as dideoxynucleotide.

The primer or the probe may be a single-stranded oligonucleotide. The primer or the probe may include deoxyribonucleotide, ribonucleotide or a combination thereof. The primers or probes used in the present disclosure may comprise naturally occurring dNMP (*i.e.*, dAMP, dGM, dCMP and dTMP), modified nucleotides, or non-natural nucleotides. A representative example of the bases of the non-natural nucleotides is inosine, known as a universal base.

The sequence of the primer or probe needs not be perfectly complementary to that of a template. So long as it allows the primer or probe to hybridize with a template to exert its own function, any complementarity is sufficient therebetween.

The term "annealing" or "priming," as used herein, refers to the apposition of an oligonucleotide or nucleic acid to a template nucleic acid, whereby the polymerase can polymerize nucleotides into a nucleic acid molecule which is complementary to the template nucleic acid or a portion thereof.

As used herein, the term, "hybridization" refers to the formation of a double-stranded nucleic acid from two single-stranded polynucleotides through non-covalent bonds between complementary nucleotide sequences under a predetermined hybridization condition.

In an embodiment, the target nucleic acid-specific oligonucleotide includes a hybridization nucleotide sequence for the target nucleic acid sequence.

As used herein, the term "a hybridizing nucleotide sequence to a target nucleic acid sequence" means "a nucleotide sequence hybridizing to a target nucleic acid sequence."

According to an embodiment, the hybridizing nucleotide sequence of an oligonucleotide includes a sequence that can hybridize with a target nucleic acid sequence in a given hybridization condition.

According to an embodiment of the present disclosure, the oligonucleotide includes a hybridizing nucleotide sequence that can hybridize with a target nucleic acid sequence in a stringent condition.

In the present disclosure, the suitable hybridization conditions or stringent conditions may be routinely determined by optimization procedures. Such procedures are routinely conducted by those skilled in the art to establish protocols for use in a laboratory. For example, conditions such as temperature, concentration of components, hybridization and washing times, buffer components, and their pH and ionic strength may be varied depending on various factors such as the length and GC content of oligonucleotide and target nucleotide sequence. For detailed conditions of hybridization, reference may be made to Joseph Sambrook, et al., Molecular Cloning, A Laboratory Manual, Cold Spring Harbor Laboratory Press, Cold Spring Harbor, N.Y. (2001); and M. L. M. Anderson, Nucleic Acid Hybridization, Springer-Verlag New York Inc. N.Y. (1999).

In an embodiment of the present disclosure, the stringent condition includes a temperature condition in which a temperature is set forth to be selected within a certain range around a Tm value of a target sequence to be hybridized by the oligonucleotide, for example, a temperature selected from among Tm value ±1°C, ±2°C, ±3°C, ±4°C, ±5°C, and ±7°C.

In an embodiment, the stringent condition may include the following conditions:
(1) 5×SSC, 5×Denhardt's solution, 0.5% SDS, 50% formamide 32 °C, (2) 5×SSC, 5×Denhardt's solution, 0.5% SDS, 50% formamide, 42 °C, (3) 5×SSC, 1% SDS, 50 mM Tris-HCl (pH 7.5), 50% formamide 42°C, (4) 5×SSC, 5×Denhardt's solution, 0.5% SDS, 50% formamide 50 °C, (5) 0.2×SSC, 0.1% SDS 60°C, (6) 0.2×SSC, 0.1% SDS 62 °C, (7) 0.2×SSC, 0.1% SDS 65 °C, or (8) 0.1 ×SSC, 0.1% SDS 65 °C, but is not limited thereto.

According to an embodiment, the hybridizing nucleotide sequence of the oligonucleotide may be determined depending on a hybridization condition employed, a nucleic acid sequence to be hybridized by the oligonucleotide, etc.

According to an embodiment, the stringent condition is a temperature at which the entire sequence of the oligonucleotide hybridizes with a target nucleic acid sequence, but not with non-target nucleic acid sequences.

According to an embodiment, the stringent condition is a temperature at which a portion (e.g., 5'- or 3'-terminal region) of the oligonucleotide sequence hybridizes with a target nucleic acid sequence, but not with non-target nucleic acid sequences.

With respect to relationship between two different oligonucleotides, the expression "include(s) (or comprise(s)) a hybridizing nucleotide sequence," as used herein, means that entirety or portion of one oligonucleotide has a complementary nucleotide sequence necessary for hybridization with entirety or portion of the other oligonucleotide.

According to an embodiment, an oligonucleotide may include a hybridizing nucleotide sequence complementary to a target nucleic acid sequence.

As used herein, the term "complementary" mean that primers or probes are complementary enough to hybridize selectively to a target nucleic acid sequence in a predetermined annealing condition or stringent condition and is intended to encompass "substantially complementary" and "perfectly complementary," particularly, perfectly complementary.

The term "substantially complementary" means that there may be 1-4 mismatches, 1-3 mismatches, or 1-2 mismatches between two oligonucleotides.

When referring to hybridization between a portion of one oligonucleotide and entirety of another oligonucleotide, the portion of one oligonucleotide may be regarded as an individual oligonucleotide.

The hybridization may occur between two nucleic acid sequences which are perfectly complementary (perfect matched) to each other or substantially complementary even in the presence of some mismatches (e.g., 1-4 mismatches, 1-3 mismatches or 1-2 mismatches) at a hybridization occurrence site (double-strand formation site) therebetween. The degree of complementarity required for hybridization may vary depending on the hybridization reaction conditions and may be controlled by, particularly, temperature.

By way of example, the degree of complementarity required for hybridization may be 70% or higher, 75% or higher, 80% or higher, 85% or higher, 90% or higher, or 95% or higher.

According to an embodiment, the degree of complementarity or the number of mismatches between two oligonucleotides is determined with reference to a site where hybridization occurs.

As used herein, the terms "hybridization" and "annealing" are not different from each other and are used interchangeably with each other.

The oligonucleotides used in the present disclosure may have various structures known in the art; for example, they may have a hairpin structure or stem-loop structure, may have a linker (spacer), or may comprise a tag that does not hybridize with a target nucleic acid.

The composition for detecting a target nucleic acid according to the present disclosure comprises a thermostable DNA polymerase. The thermostable DNA polymerase comprises thermostable DNA polymerases that can be obtained from a variety of bacterial species, for example, DNA polymerases from *Thermus aquaticus* (Taq), *Thermus thermophiles* (Tth), *Thermus filiformis, Thermis flavus, Thermococcus literalis,* or *Pyrococcus furiosus* (Pfu). Most of the DNA polymerases described above are commercially available.

The thermostable DNA polymerase may be wild-type or a mutant modified to improve its activity, etc. In one embodiment, the thermostable DNA polymerase is wild-type Taq DNA polymerase.

In one embodiment, the thermostable DNA polymerase is a DNA polymerase with improved processivity.

The processivity of a polymerase refers to the ability of the enzyme to continuously synthesize new nucleic acids by binding to the substrate (*i.e.*, DNA or RNA) used as a template without being detached therefrom during nucleic acid replication. This processivity is also called persistence.

In one embodiment, the DNA polymerase with improved processivity comprises the polymerase described in International Patent Publication No. WO2001/092501. These polymerases with improved processivity exhibit improved processivity due to the presence of a sequence-non-specific double stranded DNA binding domain bound to the polymerase or the enzymatic domain of the polymerase.

In one embodiment, the sequence-non-specific double stranded DNA binding domain may be Sso7d or Sac7d protein of about 7,000 kD MW derived from thermotolerant organisms, for example, hyperthermophilic archaea *Sulfolobus solfataricus* and *S. acidocaldarius* (see WO 2001/092501).

In one embodiment, the composition for detecting a target nucleic acid comprises a thermostable DNA polymerase, which is an Sso7d fusion DNA polymerase, where Sso7d is fused.

In the Sso7d fusion DNA polymerase described above, the Sso7d may be fused in various ways as long as it does not affect the activity or function of the DNA polymerase. In one embodiment, the Sso7d is linked to the N-terminus of the amino acid sequence of DNA polymerase. In another embodiment, the Sso7d is linked to the C-terminus of the amino acid sequence of DNA polymerase. In still another embodiment, the Sso7d is linked to the inside of the amino acid sequence of DNA polymerase.

As used herein, the term "reverse transcriptase," which is also called RNA-dependent DNA polymerase, refers to an enzyme that uses RNA as a template and synthesizes complementary DNA.

In order to detect RNA viruses, a reverse transcription reaction to generate cDNA from an RNA template is essential. The specific details are disclosed in Joseph

Sambrook, et al., Molecular Cloning, A Laboratory Manual, Cold Spring Harbor Laboratory Press, Cold Spring Harbor, N.Y. (2001); and Noonan, K. F. et al., Nucleic Acids Res. 16:10366 (1988).

In one embodiment, as the reverse transcriptase, reverse transcriptases originating from a variety of sources may be used, for example, avian myeloblastosis virus-derived reverse transcriptase (AMV RTase), murine leukemia virus-derived reverse transcriptase (MuLV RTase) and Rous-associated virus 2 reverse transcriptase (RAV-2 RTase) may be used, but the reverse transcriptase is not limited thereto.

A reverse transcriptase requires a primer to synthesize cDNA from an RNA template. There are three main types of primers used in reverse transcription reactions: (i) an oligo dT primer that is annealed to the poly A tail of mRNA and synthesizes cDNA from the 3'-end, (ii) a random primer made of random nucleotides of 6 nt to 9 nt in size to initiate synthesis in all regions of RNA; and (iii) a target-specific primer that synthesizes only target cDNA.

According to one embodiment, the composition may comprise a primer for a reverse transcription reaction, and the primer for the reverse transcription reaction may be a target-specific primer.

In one embodiment, the target-specific primer for the reverse transcription reaction is used to synthesize cDNA in the reverse transcription reaction, and subsequently, it may be used as a primer pair for the amplification of the obtained cDNA by pairing with another primer (*e.g.*, a forward primer or a reverse primer) in the amplification reaction of the target nucleic acid sequence.

In one embodiment, the nucleic acid amplification components further comprise UDG.

In the case of a reverse transcription reaction or nucleic acid amplification reaction, carry-over contamination may occur where the previous reaction product(s) (*e.g.*, cDNA or an amplicon(s)) contaminate(s) the reaction to be newly performed through various routes. That is, there may be a problem in that the carry-over contaminant may act as a template in the reaction to be newly performed and may thus be reverse-transcribed or amplified thus showing a false positive result as if it were positive even when it is actually negative.

Such carry-over contamination may be prevented by using uracil DNA glycosylase (UDG). Specifically, among dNTPs, dUTP is used instead of dTTP to perform a reverse transcription reaction or amplification reaction. The resulting products (*i.e.*, cDNA or amplicons) comprise dUTP, and previous products including dUTP are hydrolyzed by treating with UDG before performing the new reaction. As a result, carry-over contamination can be prevented.

As an example of a suitable UDG, heat labile uracil DNA glycosylase (UDG) may be used. The heat labile UDG may comprise UDG derived from cryophilic organisms, for example, UDG derived from psychrophilic bacteria or Alaskan cod, but is not limited thereto. These enzymes have the characteristic of being quickly and irreversibly inactivated when exposed to a temperature of 50°C or 55°C, respectively. Therefore, the heat labile UDG is inactivated before the reverse transcription reaction begins, so that only carry-over contaminants can be removed without affecting the cDNA generation process by the reverse transcription reaction.

In one embodiment, the nucleic acid amplification components further comprise dNTPs. The dNTPs, which refer to nucleoside triphosphates that constitute nucleic acids, consist of dATP, dGTP, dCTP, dUTP, and dTTP. The five types of nucleoside triphosphates may be present in the same amount in the composition for detecting a target nucleic acid.

Further, the dNTPs may be comprised in a buffer. the buffer comprises Tris buffer of pH 7 to pH 8.

Typical reaction mixtures for amplifying nucleic acids present in samples include a buffer in an amount such that the buffer is present at a concentration of 20 mM to 50 mM. In contrast, the buffer comprising dNTP of the present disclosure includes Tris buffer in an amount that results in a relatively high concentration of 60 mM to 120 mM in the reaction mixture. This relatively high concentration of Tris buffer exhibits the effect of improving the detection specificity and signal intensity of the target nucleic acid.

In one embodiment, the Tris buffer is comprised in the reaction mixture to have a concentration of 60 mM to 120 mM, and specifically, it may be at a concentration of 70 mM to 120 mM, 80 mM to 120 mM, 60 mM to 110 mM, 70 mM to 110 mM, or 80 mM to 110 mM.

In an embodiment, the nucleic acid amplification components (i) to (iii) are contained in separate containers. The nucleic acid amplification components can be accommodated in individual tubes, cartridges, or multi-well plates. To prepare the reagent for nucleic acid amplification, the first pipetting mechanism may dispense each reagent to the second plate from a container containing each component for nucleic acid amplification. Meanwhile, other components related to the nucleic acid amplification reaction, such as stabilizers and co-factors, may be included in the containers of (i) to (iii) or in separate containers.

### Step (d): Dispensing Nucleic Acids Using the Second Pipetting Mechanism

In this step, the nucleic acids extracted from the first plate are dispensed into the second plate containing the nucleic acid amplification reagents using the second pipetting mechanism.

In an embodiment, the second plate is a 96 well plate. Once nucleic acid extraction is completed at the first plate in step (b), the nucleic acids are dispensed into the second plate, where the nucleic acid amplification reagents have been dispensed, completing the setup for the nucleic acid amplification reaction. At this time, if the first plate and the second plate are configured as plates containing 96 wells, the extracted nucleic acids can be dispensed into each well of the second plate at once using the second pipetting mechanism. For example, step (d) can be performed with a second pipetting mechanism equipped with pipette tips having a capacity of 200 to 400 µL, 250 to 350 µL, or approximately 300 µL. Thus, a large number of samples can be processed simultaneously.

In an embodiment, the preparation of the nucleic acid extraction and amplification reaction mixture by the method is performed in a time reduced by 30% to 80%, 50% to 80%, 60% to 80%, or 70% to 80% compared to the time required when performed in a sample handling system that includes only the first pipetting mechanism. The nucleic acid amplification reaction setup method of the present invention significantly reduces the time required for the nucleic acid extraction process, particularly by decreasing the time and types of nucleic acid extraction reagents dispensed for the sample.

For example, a typical nucleic acid amplification reaction setup takes a total of about 2.5 hours or more, or more than 3 hours. In contrast, the method for preparing a reaction mixture for nucleic acid amplification according to the present invention takes a total of about 1.5 hours or less, 1 hour or less, 50 minutes or less, or 45 minutes or less. This is achieved as a synergistic effect resulting from the comprehensive action of the driving methods of the pipetting mechanism applied to the above sample handling system, various types of consumables such as plates, and the composition of reagents, and ultimately can improve the efficiency and usability of all molecular diagnostic methods using nucleic acid amplification reactions.

In another aspect of the invention, the present disclosure provides a sample handling system for preparing reaction mixtures for amplification reaction of nucleic acids in samples, comprising a first pipetting mechanism including 4 to 8 pipetting channels for aspirating and dispensing metered liquids, a second pipetting mechanism including 96 pipetting channels for aspirating and dispensing metered liquids, a section for mounting a first plate having 96 wells, a section for mounting a second plate having 96 wells, a section for nucleic acid extraction, a section for applying magnetic field, a section for mounting sample vessel, a section for mounting a nucleic acid extraction reagent vessel, a section for mounting a nucleic acid amplification component vessel, a section for mounting pipette tips for the pipetting channels of the first pipetting mechanism and a section for mounting pipette tips for the pipetting channels of the second pipetting mechanism, wherein the first pipetting mechanism is configured to move the 4 to 8 pipetting channels independently, and the second pipetting mechanism is configured to move all 96 pipetting channels together, wherein each pipetting channel has a pipette tip mounted or mountable thereon.

In an embodiment, the first pipetting mechanism and the second pipetting mechanism are located separately within the sample handling system.

In an embodiment, the first pipetting mechanism is configured to move 4 to 8 pipetting channels in a direction of X-axis simultaneously, and to move them in directions of Y-axis and Z-axis independently.

In an embodiment, the sample handling system further comprises plate grippers configured to move the first plate and the second plate from one section to another.

In an embodiment, the plate grippers are mounted on two pipetting channels of the first pipetting mechanism, the two pipetting channels being arranged on opposite sides of the plate respectively such that the plate grippers grip and move the plate.

In an embodiment, the second pipetting mechanism is configured to dispense the nucleic acid extraction reagent into each well of the first plate and dispense the extracted nucleic acid into each well of the second plate.

The sample handling system of the present disclosure is intended to perform the method for preparing a reaction mixture for nucleic acid amplification within a sample using the aforementioned sample handling system described above, and the descriptions regarding common contents between the two are omitted herein in order to avoid excessive complexity of the present specification.

The present invention will now be described in further detail by examples. It would be obvious to those skilled in the art that these examples are intended to be more concretely illustrative and the scope of the present invention as set forth in the appended claims is not limited to or by the examples.

### [Mode for Invention]

### PREPARATION

### 1. Preparation of Smples

To prepare the SARS-CoV-2 samples, plasmid DNA containing the sequences of the E gene, RdRP gene, and N gene of SARS-CoV-2 (Wild type) as the RNA target nucleic acid, along with the T7 promoter sequence, was synthesized by IDT. Subsequently, SARS-CoV-2 E gene template, RdRP gene template, and N gene template were prepared by in vitro transcription using T7 RNA polymerase.

Afterward, samples were prepared by adding the above prepared gene templates to negative clinical specimens stored at -80°C. The negative clinical specimens were collected from healthy individuals using a specimen collection kit, with nasopharyngeal and oropharyngeal (throat) swabs stored together in a single UTM (Universal Transport Medium, COPAN, 360C) and kept at -80°C.

ACTB (GenBank ID: NM_001101.2) was used as an internal control. For this purpose, Total RNA Control (Human) (Applied Biosystems, Cat. No. 4307281), extracted from cells, was used as the internal control template.

### 2. Preparation of Reagent for Amplifying SARS-CoV-2 Nucleic Acids

To detect the target nucleic acids of SARS-CoV-2, including the E gene, RdRP gene, and N gene, 5 µL of oligonucleotides capable of hybridizing with the target nucleic acids of SARS-CoV-2, 5 µL of enzyme mix (Sso7d-Taq DNA polymerase 2 U, RTase 2 U, UDG 0.1 U), 0.8 mM hot-start dNTP, and 5 µL of buffer (including 30 mM MgCl2, 400 mM Tris-HCl, etc.) were prepared in each tube for mixing.

The oligonucleotides capable of hybridizing with the SARS-CoV-2 target nucleic acids were derived from the Allplex^{™} SARS-CoV-2 Master Assay (Cat. No. RV10284X, Seegene Inc.), a multiplex one-step RT-PCR product capable of detecting multiple targets in a single tube. The RTase was sourced from ThermoFisher (Cat. No. 18080044), and UDG was obtained from Enzynomics (Cat. No. M013).

### EXAMPLES

### 1. Preparation of Reaction Mixtures for Nucleic Acid Amplification

### < Comparative Example>

5 µL of proteinase K was added to an empty tube and mixed with 40 µL of distilled water, resulting in a final volume of 45 µL with a proteinase K concentration of 2.22 mg/mL. Then, after thawing the sample stored at -80°C from Preparation 1 at room temperature, 15 µL of transport medium containing the sample was dispensed into each tube containing proteinase K. The tubes were placed in a real-time thermal cycler (CFX96, Bio-Rad) and incubated at 98°C for 5 minutes, followed by cooling at 4°C for 3 minutes to prepare a crude extract sample. Afterward, an automated extraction system (Microlab STARlet IVD, Hamilton) equipped with 8 pipetting channels was used to extract nucleic acids by mounting the prepared samples and an extraction kit (STARMag 96 X 4 Universal Cartridge Kit, Seegene).

The extraction kit contained Lysis Buffer, Binding Buffer, Wash Buffer 1, Wash Buffer 2, Wash Buffer 3, Elution Buffer, and Magnetic Beads, each provided in separate cartridges.

After nucleic acid extraction, the nucleic acid amplification reagents from Preparation 2 and the extracted nucleic acids were mixed within the system to prepare the reaction mixture, completing the nucleic acid amplification reaction setup for a total of 8 tubes. The total time required for preparing the nucleic acid amplification reaction mixture was 2 hours and 50 minutes.

### <Example>

The automated extraction system equipped with a first pipetting mechanism containing 8 pipetting channels and a second pipetting mechanism containing 96 pipetting channels was used to extract nucleic acids by mounting the extraction kit and each sample from Preparation 1.

The extraction kit consisted of a 96 deep-well plate preloaded with magnetic beads, a 96-well plate preloaded with lysis/binding buffer, the first wash buffer, the second wash buffer, and elution buffer. The lysis/binding buffer included PEG 8000 and N-lauroyl sarcosine but did not contain proteinase K. The first wash buffer contained guanidine hydrochloride, the second wash buffer was 82% ethanol (EtOH), and the elution buffer was ultrapure water (UPW).

The automated extraction system performed a nucleic acid extraction process by dispensing a sample onto a bead plate filled with beads using a first pipetting mechanism, and then aspirating/dispensing the same reagent from the buffer plates of the extraction kit to all samples at once using a second pipetting mechanism. After dispensing the elution buffer, incubation was performed for 10 minutes or more. In addition, each reagent of Preparation 2 was mixed into a second plate using the first pipetting mechanism to prepare a nucleic acid amplification reagent.

After completing nucleic acid extraction in the first plate, the second pipetting mechanism dispensed the extracted nucleic acids from the first plate into the second plate, which contained the nucleic acid amplification reagents, to complete the nucleic acid amplification reaction setup for a total of 8 tubes. The preparation time for the nucleic acid amplification reaction mixture was 55 minutes.

### 2. One-step RT-PCR

The tubes containing the reaction mixtures prepared in the above comparative example and the example were placed in a real-time thermal cycler (CFX96, Bio-Rad) and incubated at 50°C for 10 minutes, followed by denaturation at 95°C for 3 minutes, and cycled 5 times with steps of 95°C for 5 seconds and 60°C for 20 seconds, and then cycled 40 times at 95°C for 2 seconds and 60°C for 5 seconds. Signal detection was performed at 60°C in each cycle. The signals detected at the above temperature were analyzed using the Seegene viewer software. The experiment was repeated twice for each preparation to obtain the average Ct values (Table 1).

**[Table 1] average Ct value**

| Sample (Copies/rxn) | | Comparative Example | | | Example | | |
|---|---|---|---|---|---|---|---|
| | | E gene | RdRP gene | N gene | E gene | RDRP gene | N gene |
| 1 | 10^3 | 24.07 | 24.70 | 23.58 | 24.50 | 25.77 | 24.90 |
| | 10^2 | 28.11 | 28.52 | 27.02 | 28.28 | 29.12 | 28.55 |
| 2 | 10^3 | 23.84 | 24.62 | 23.76 | 24.65 | 26.05 | 25.55 |
| | 10^2 | 27.68 | 28.14 | 26.78 | 27.84 | 29.88 | 27.82 |
| 3 | 10^3 | 24.06 | 25.07 | 24.41 | 24.15 | 25.33 | 24.80 |
| | 10^2 | 27.82 | 29.03 | 28.35 | 28.10 | 29.15 | 28.88 |
| 4 | 10^3 | 23.90 | 25.31 | 23.95 | 23.95 | 25.74 | 24.35 |
| | 10^2 | 28.28 | 28.31 | 26.90 | 28.35 | 28.65 | 27.45 |

As shown in [Table 1], it was confirmed that the nucleic acid amplification reaction mixture (Example) prepared using the method according to the present disclosure enabled stable detection at a level equivalent to the reaction mixture for nucleic acid amplification prepared by extracting the target nucleic acid using a conventional method (Comparative Example). Considering that the time required to prepare the reaction mixture for the nucleic acid amplification reaction in the Example was shortened by about 1 hour and 50 minutes compared to the Comparative Example, it is evident that the method of preparing the reaction mixture for the nucleic acid amplification reaction according to the present invention is highly efficient.

Therefore, by using the method of the present invention, the nucleic acid amplification reaction mixture can be prepared in a very short time, and when applied for detecting target nucleic acids, it allows faster results while ensuring high accuracy due to the nature of molecular diagnostics.

## Claims

1. A method for preparing reaction mixtures for amplifying nucleic acids present in samples using a sample handling system,
wherein the sample handling system comprises a first pipetting mechanism including 4 to 8 pipetting channels for aspirating and dispensing metered liquids, and a second pipetting mechanism including 96 pipetting channels for aspirating and dispensing metered liquids, wherein the first pipetting mechanism is configured to move the 4 to 8 pipetting channels independently, and the second pipetting mechanism is configured to move all 96 pipetting channels together, wherein each pipetting channel has a pipette tip mounted or mountable thereon,
wherein the method comprises:
(a) dispensing a plurality of samples into each well of a first plate having 96 wells using the first pipetting mechanism;
(b) extracting nucleic acids from the samples by dispensing a nucleic acid extraction reagent into each well of the first plate using the second pipetting mechanism;
(c) preparing a nucleic acid amplification reagent by combining nucleic acid amplification components, and then dispensing the nucleic acid amplification reagent into each well of a second plate having 96 wells using the first pipetting mechanism; and
(d) dispensing nucleic acids extracted in step (b) into each well of the second plate by using the second pipetting mechanism.

2. The method according to claim 1, wherein the first pipetting mechanism and the second pipetting mechanism are located separately within the sample handling system.

3. The method according to claim 1, wherein the first pipetting mechanism is configured to move 4 to 8 pipetting channels in a direction of X-axis simultaneously, and to move them in directions of Y-axis and Z-axis independently.

4. The method according to claim 1, wherein the second pipetting mechanism is configured to move 96 pipetting channels in the direction of Z-axis independently.

5. The method according to claim 1, wherein the sample handling system comprises:
a section for mounting the first plate;
a section for mounting the second plate;
a section for nucleic acid extraction; and
a section for applying magnetic field.

6. The method according to claim 1, wherein the sample handling system further comprises:
a section for mounting sample vessel;
a section for mounting a nucleic acid extraction reagent vessel; and
a section for mounting a nucleic acid amplification component vessel.

7. The method according to claim 1, wherein the sample handling system further comprises:
a section for mounting pipette tips for the pipetting channels of the first pipetting mechanism; and
a section for mounting pipette tips for the pipetting channels of the second pipetting mechanism.

8. The method according to claim 7, wherein the sections for mounting pipette tips each comprise:
a section equipped with pipette tips having a capacity of 200 to 400 µL; and
a section equipped with pipette tips having a capacity of 900 to 1,100 µL.

9. The method according to claim 1, wherein the sample handling system further comprises plate grippers configured to move the first plate and the second plate from one section to another.

10. The method according to claim 9, wherein the plate grippers are mounted on two pipetting channels of the first pipetting mechanism, the two pipetting channels being arranged on opposite sides of the plate respectively such that the plate grippers grip and move the plate.

11. The method according to claim 1, wherein, in step (a), the internal control (IC) is dispensed using the first pipetting mechanism equipped with a pipette tip having a capacity of 200 to 400 µL.

12. The method according to claim 1, wherein the nucleic acid extraction reagent comprises a lysis/binding buffer, a first wash buffer, a second wash buffer, and an elution buffer, each contained in a separate vessel.

13. The method according to claim 12, wherein, in step (a), the first plate is preloaded with magnetic beads, and the sample is dispensed after mounting the first plate in the section for nucleic acid extraction.

14. The method according to claim 13, wherein, in step (b), the method comprises:
transferring the first plate to the section for applying magnetic field after dispensing 300 to 500 µL of the lysis/binding buffer into the first plate;
removing the lysate using the second pipetting mechanism after collecting the beads by means of the magnetic field; and
transferring the first plate with the lysate removed to the section for nucleic acid extraction.

15. The method according to claim 1, wherein the nucleic acid extraction reagent comprises proteinase K, a lysis buffer, a binding buffer, a first wash buffer, a second wash buffer, a third wash buffer and elution buffer.

16. The method according to claim 15, wherein the sample handling system further comprises a section for mounting a magnetic bead vessel.

17. The method according to claim 15, wherein, in step (a), before dispensing the sample, the first plate is mounted on the section for nucleic acids extraction, followed by dispensing 3 to 20 µL of proteinase K into the first plate.

18. The method according to claim 17, wherein the proteinase K is dispensed using the second pipetting mechanism equipped with the pipette tip having a capacity of 200 to 400 µL.

19. The method according to claim 17, wherein, in step (b), the method comprises:
dispensing 100 to 200 µL of lysis buffer into the first plate;
dispensing 3 to 20 µL of magnetic beads into the first plate;
dispensing 500 to 700 µL of binding buffer, followed by transferring the plate to the section for applying magnetic field;
removing the lysate using the second pipetting mechanism after collecting the beads by means of the magnetic field; and
transferring the first plate with the lysate removed to the section for nucleic acids extraction.

20. The method according to claim 12 or 15, wherein, in step (b), the method comprises:
dispensing 400 to 600 µL of the first wash buffer into the first plate, followed by transferring the first plate to the section for nucleic acid extraction.
shaking the first plate in the section for nucleic acid extraction;
transferring the first plate to the section for applying magnetic field;
removing the first wash buffer using the second pipetting mechanism after collecting the beads by means of the magnetic field; and
transferring the first plate from which the first wash buffer has been removed to the section for nucleic acid extraction

21. The method according to claim 12 or 15, wherein, in step (b), the second wash buffer and/or the third wash buffer are applied in the same manner as the first wash buffer, and the method comprises:
transferring the first plate from which the second wash buffer and/or the third wash buffer have been removed to the section for nucleic acid extraction, followed by incubating the first plate.

22. The method according to claim 12 or 15, wherein, in step (b), the method comprises:
dispensing 50 to 200 µL of the elution buffer into the first plate, followed by shaking the first plate.

23. The method according to claim 1, wherein the first plate having 96 wells is a 96 deep-well plate.

24. the method according to claim 1, wherein the section for nucleic acid extraction is configured to perform any one of the processes selected from the group consisting of stirring, shaking, ultrasonication, vortexing, thermal treatment, pipetting and combinations thereof.

25. The method according to claim 6, wherein the nucleic acid extraction reagent vessel is a 96 deep-well plate or a reservoir plate having a single well.

26. The method according to claim 1, wherein the second plate is a 96 well plate.

27. The method according to claim 1, wherein the step (c) is performed before, concurrently with, or after step (a).

28. The method according to claim 1, wherein the nucleic acid amplification components comprise (i) an oligonucleotide set specific to a target nucleic acid, (ii) DNA polymerases, and optionally reverse transcriptase (RTase) or uracil-DNA glycosylase (UDG), and (iii) dNTP.

29. The method according to claim 1, wherein the step (d) is performed by the second pipetting mechanism equipped with the pipette tip having a capacity of 200 to 400 µL.

30. The method according to claim 1, wherein the preparation of the nucleic acid extraction and amplification reaction mixture by the method is performed in a time reduced by 30 to 80% compared to the time required when performed in a sample handling system that includes only the first pipetting mechanism.

31. A sample handling system for preparing reaction mixtures for amplification reaction of nucleic acids in samples, comprising:
a first pipetting mechanism including 4 to 8 pipetting channels for aspirating and dispensing metered liquids;
a second pipetting mechanism including 96 pipetting channels for aspirating and dispensing metered liquids;
a section for mounting a first plate having 96 wells;
a section for mounting a second plate having 96 wells;
a section for nucleic acid extraction;
a section for applying magnetic field;
a section for mounting sample vessel;
a section for mounting a nucleic acid extraction reagent vessel;
a section for mounting a nucleic acid amplification component vessel;
a section for mounting pipette tips for the pipetting channels of the first pipetting mechanism; and
a section for mounting pipette tips for the pipetting channels of the second pipetting mechanism,
wherein the first pipetting mechanism is configured to move the 4 to 8 pipetting channels independently, and the second pipetting mechanism is configured to move all 96 pipetting channels together, wherein each pipetting channel has a pipette tip mounted or mountable thereon.

32. The system according to claim 31, wherein the first pipetting mechanism and the second pipetting mechanism are located separately within the sample handling system.

33. The system according to claim 31, wherein the first pipetting mechanism is configured to move 4 to 8 pipetting channels in a direction of X-axis simultaneously, and to move them in directions of Y-axis and Z-axis independently.

34. The system according to claim 31, wherein the sample handling system further comprises plate grippers configured to move the first plate and the second plate from one section to another,
wherein the plate grippers are mounted on two pipetting channels of the first pipetting mechanism, the two pipetting channels being arranged on opposite sides of the plate respectively such that the plate grippers grip and move the plate.

35. The system according to claim 31, wherein the second pipetting mechanism is configured to dispense the nucleic acid extraction reagent into each well of the first plate and dispense the extracted nucleic acid into each well of the second plate.
